# EUROPEAN PATENT SPECIFICATION

(11) **EP 2 986 263 B1**
(45) Date of publication and mention of the grant of the patent: **11.01.2017**
(21) Application number: 14727919.4
(22) Date of filing: 17.04.2014
(51) Int. Cl.: A61F 13/15, A61F 13/532, A61F 13/534, A61F 13/539

(54) **MACHINE FOR MAKING AN ABSORBENT SANITARY ARTICLE**
MASCHINE ZUR HERSTELLUNG EINES ABSORBIERENDEN ARTIKELS
MACHINE POUR FABRIQUER UN ARTICLE HYGIÉNIQUE ABSORBANT

(30) Priority: 18.04.2013 IT BO20130176
(43) Date of publication of application: 24.02.2016
(73) Proprietor: GDM S.p.A., 40133 Bologna (IT)
(72) Inventor: PIANTONI, Matteo, 24021 ALBINO (Bergamo) (IT); SOLI, Valerio, 40135 Bologna (IT)
(74) Representative: Bianciardi, Ezio
(86) International application number: PCT/IB2014/060800
(87) International publication number: WO 2014/170859

(56) References cited:
- WO-A1-2011/080858
- WO-A1-2012/048878
- US-A1- 2013 079 741

## Description

### Technical field

This invention relates to a machine for making an absorbent sanitary article, such as a baby nappy or the like, and to a machine for making articles of this kind.

In this description, for simplicity, express reference is made to a nappy but without thereby limiting the scope of the invention.

### Background art

As is known, nappies comprise an absorbent pad or padding which is normally enclosed between a permeable inner layer of non-woven fabric and an impermeable outer layer of polyethylene.

Absorbent pads of known type comprise an absorbent core made of an absorbent material, such as, for example, granules of superabsorbent polymer material (SAP) inside a mixture of containment cellulose pulp (fluff) and absorbent material binder, sandwiched between two layers of non-woven fabric.

In order to improve drainage of the absorbed liquid and, at the same time, reduce production costs, manufacturers are opting for absorbent cores with more and more granular superabsorbent material and less and less cellulose pulp, and even absorbent cores that are totally free of cellulose. One example of absorbent pads comprising absorbent cores made of superabsorbent polymer material and substantially free of cellulose is described in document EP2444046.

The padding described in that prior art document comprises a first layer of non-woven fabric, a second layer of non-woven fabric and the cellulose free absorbent material enclosed between the two layers of non-woven fabric. The two layers of non-woven fabric are joined by seals which are suitably made in such a way as to form a plurality of cells containing the absorbent material.

In one embodiment, the seals which separate the cells are uninterrupted so as to better contain and position the absorbent material, especially when the product is new, unused and the superabsorbent material dry.

In a second embodiment, the seals have suitable breaks in them so as to improve the diffusion of the liquid and optimize its absorption by the absorbent material.

These solutions, where the absorbent core is cellulose free and the padding thus less coherent, have some disadvantages, however.

In the first of the above mentioned embodiments, the seals compress the absorbent material, limiting its freedom of expansion and thus its absorbing capacity.

If relatively large quantities of liquid are poured into the nappy, the absorbent material may increase in volume until it tears the seals and migrates into an adjacent cell, thus altering its optimal distribution within the pad.

In the second embodiment, the breaks in the seals may allow the granules of absorbent material to move through them relative to their optimal distribution, even when dry.

In the second embodiment, too, although the breaks in the seals provide preferential channels through which the absorbent material may spread, excessive amounts of absorbed liquid may cause the seals themselves to tear, leading to uncontrolled movement of the absorbent material within the pad.

In this context, the main technical purpose of this invention is to propose an absorbent sanitary article which is free of the above mentioned disadvantages.

### Aim of the invention

The aim of this invention is to propose an absorbent sanitary article whose padding is substantially free of cellulose and where the absorbent material is allowed to spread in a suitably controlled manner.

The technical purpose and aims specified are substantially achieved by a machine for making an absorbent sanitary article according to claim 1.

### Brief description of the drawings

Further features and advantages of this invention are more apparent in the description below, with reference to preferred, non-exclusive embodiments of an absorbent sanitary article and of a machine for making the absorbent sanitary article, as illustrated in the accompanying drawings, in which:
- Figure 1 is a schematic cross section illustrating an absorbent sanitary article produced by a machine according to this invention;
- Figure 2 is a schematic cross section illustrating a first embodiment of the absorbent sanitary article produced by a machine according to this invention;
- Figure 3 is a schematic cross section, with some parts cut away for better clarity, illustrating a second embodiment of the absorbent sanitary article produced by a machine according to this invention;
- Figure 4 is a schematic cross section, with some parts cut away for better clarity, illustrating a third embodiment of the absorbent sanitary article produced by a machine according to this invention;
- Figure 5 is a schematic plan view, with some parts cut away for better clarity, illustrating an absorbent sanitary article produced by a machine according to this invention;
- Figures 6 to 9 are schematic views, partly in blocks and with some parts cut away in order to better illustrate others, showing different embodiments of an installation for making an absorbent sanitary article according to the invention.

### Detailed description of preferred embodiments of the invention

With reference to the accompanying drawings, the numeral 1 denotes an absorbent sanitary article, only partly illustrated, such as for example, a baby nappy, to which express reference is hereinafter made without thereby limiting the scope of the invention, sanitary towels or the like. The article 1 has a main direction of extension D, a front portion and a rear portion.

In a preferred embodiment, the main extension is the longitudinal one, that is the extension measured between the front portion and the rear portion of the article 1.

The article 1 comprises an inner layer 2 permeable to liquids, also known as "top sheet", preferably defined by a piece of non-woven fabric, and an outer layer 3 impermeable to liquids, also known as "back sheet", preferably defined by a sheet of polyethylene.

The top sheet and the back sheet are joined to each other in known manner to define the outer container of the article 1.

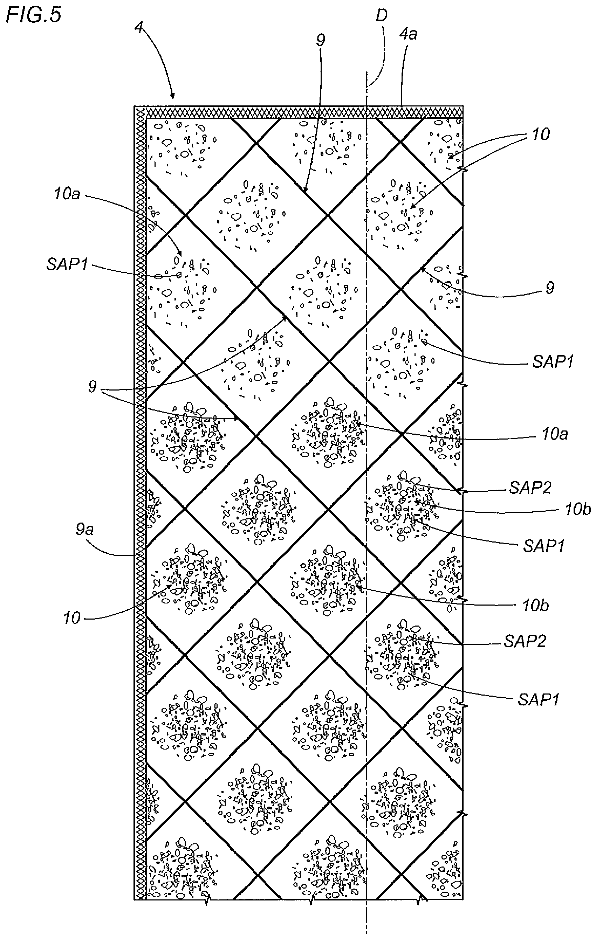

The article 1 comprises an absorbent pad 4 interposed between the permeable inner layer 2 and the impermeable outer layer 3 and delimited by an outer perimeter 4a, illustrated partly in Figure 5.

The pad 4 comprises a first layer NW1 of non-woven fabric and a second layer NW2 of non-woven fabric at least partly joined to the first layer, as explained in more detail below.

The pad 4 comprises a core 5 interposed between the first layer NW1 and the second layer NW2.

In the preferred embodiment illustrated, the core 5 comprises a spread 6 of absorbent material, preferably a polymer known as superabsorbent polymer or SAP, to which express reference is made herein without thereby limiting the invention and which is not further described.

More specifically, in one embodiment, the spread 6 of absorbent polymer material comprises at least a first absorbent polymer material SAP1.

In one embodiment, the spread 6 of absorbent polymer material comprises a first absorbent polymer material SAP1 and a second absorbent polymer material SAP2.

Advantageously, the response time to liquid of the second material SAP2 is, generally speaking, longer than the response time to liquid of the first material SAP1.

As is known, the response time to liquid is a specific property of the superabsorbent material.

The response time to liquid may, for example, be a function of the permeability and/or liquid retaining capacity and/or *absorption* and/or *retention* and/or *absorption against pressure* of the superabsorbent polymer material. In short, the performance of superabsorbent polymer materials is determined mainly, in substantially known manner, by: *permeability coefficient*, *apparent specific gravity (bulk density)*, *flow rate, centrifuge retention capacity (CRC)* and *particle size distribution (PSD).*

These properties are preferably determined and identified according to the *Worldwide Strategic Partners (WSP) test methods.*

In other words, the first material SAP1 starts absorbing liquid before the second material SAP2 which starts absorbing the liquid at a later stage. That way, during the initial stages of absorption, the second absorbent material SAP2 is permeable to the liquid which is thus free to reach the first absorbent material SAP1.

The core 5, that is, the part of the pad 4 between the layers NW1 and NW2, comprises a third layer 7 of non-woven fabric which is also interposed between the first and second layers NW1 and NW2 of non-woven fabric.

Advantageously, the layer 7 of non-woven fabric is defined by a piece of a continuous web 8 of non-woven fabric.

In one embodiment, the layer 7 extends substantially for the full length of the pad 4.

In a different embodiment, the layer 7 of non-woven fabric is not continuous along the main direction, that is to say, for each pad 4, it is composed of two or more pieces of the web 8.

In one embodiment, the layer 7 is as long as the entire pad 4 or, alternatively, is shorter than the pad 4 and preferably located in a median portion of the pad along the main direction of extension D.

Preferably, the layer 7 is made of a non-woven fabric whose grammage (*Grams per Square Metre, GSM*) is much greater than the grammage of the non-woven fabric used for the layers NW1 and NW2.

For example, the grammage of the layers NW1 and NW2 is between 8 gsm and 20 gsm, whilst the grammage of the layer 7 may be up to 400 gsm.

A preferred grammage for the layer 7 is between 80 gsm and 160 gsm, in particular equal to 120 gsm.

Advantageously, also, the fibres of the layer 7 are much more open than the fibres of the layers NW1 and NW2.

The layer 7 is preferably made from a non-woven fabric known as "high loft", which is an extruded non-woven fabric of substantially known type and composed typically of polypropylene and polyethylene, to which express reference is made herein without thereby limiting the invention. Advantageously, the high loft layer 7 defines, in the core 5, a buffer layer where the liquid remains until the absorbent material SAP1 and/or SAP2 absorbs the liquid.

The absorbent sanitary article 1 comprises connecting means operating between the first layer NW1 of non-woven fabric, the second layer NW2 of non-woven fabric and the third layer 7 of non-woven fabric in order to join them and to define the pad 4.

As schematically illustrated also in Figure 5, the connecting means comprise a plurality of seals 9.

The layers NW1, NW2 and 7 are interconnected by the seals 9.

In one embodiment, the connecting means comprise a perimeter seal 9a along the perimeter 4a of the pad 4, which prevents the absorbent material SAP1 and/or SAP2 from leaking out.

The perimeter seal 9a runs preferably along the longitudinal edges of the pad 4.

In further embodiments, the perimeter seal 9a runs preferably also along the transversal edges of the pad 4.

In the embodiment illustrated, the seals 9 are uninterrupted, whilst in alternative embodiments not illustrated, the seals 9 have breaks in them. The seals 9 define in the pad 4 a plurality of cells 10 each containing a part of the spread 6 of absorbent material.

In one embodiment, all the cells 10 contain the same absorbent material, for example SAP1 or SAP2, preferably in the same quantity.

In one embodiment, illustrated in Figure 5, the pad 4 comprises cells 10a filled with the first absorbent material SAP1 and cells 10b containing the first and the second absorbent material SAP1 and SAP2.

Preferably, the cells 10b are located in a median portion of the pad 4 along the main direction of extension D of the pad 4 itself.

Preferably, the cells 10b are located more towards the front portion of the article 1 and away from the rear portion.

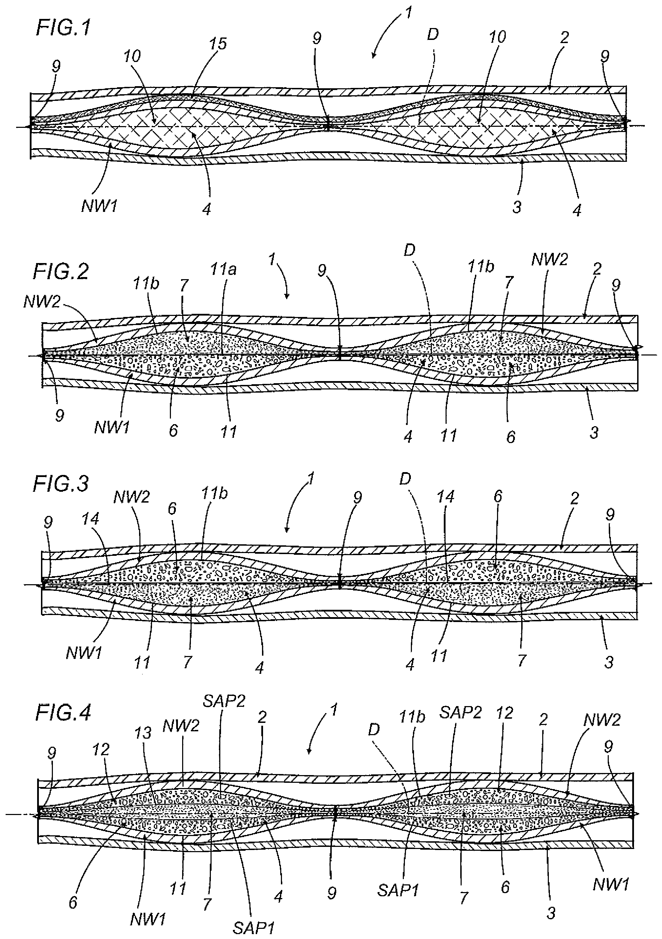

Preferably, the cells 10b contain a larger quantity of absorbent material SAP1 and/or SAP2 for better absorption in the central zone of the article 1. As illustrated in Figure 2, in a first embodiment of the article 1, the layer NW1 is located on the impermeable outer layer 3.

The spread 6 of absorbent polymer material is located on the layer NW1 of non-woven fabric.

Preferably, the pad 4 comprises an adhesive layer 11 between the layer NW1 of non-woven fabric and the spread 6 of absorbent polymer material. The layer 7 of high loft non-woven fabric is located on the spread 6 of absorbent material.

In one embodiment, the pad 4 comprises an adhesive layer 11 a between the layer 7 and the spread 6 of absorbent polymer material.

The layer NW2 is located on the high loft layer 7 and the permeable inner layer 2 is positioned on the layer NW2.

In one embodiment, the pad 4 comprises an adhesive layer 11 b between the layer 7 and the layer NW2.

With reference to Figure 4, in one embodiment of the article 1, the layer NW1 is located on the impermeable outer layer 3.

The spread 6 of absorbent polymer material is located on the layer NW1 of non-woven fabric and, preferably, the adhesive layer 11 is interposed between the layer NW1 and the spread 6.

The layer 7 of high loft non-woven fabric is located on the spread 6 of absorbent material.

The embodiment illustrated comprises a second spread 12 of absorbent material located on the high loft layer 7.

In one embodiment, the pad 4 comprises an adhesive layer 16 between the high loft layer 7 and the spread 12 of absorbent polymer material.

The second layer NW2 is located on the spread 12 to close the pad 4. Preferably, the pad 4 comprises the adhesive layer 11b between the high loft layer 7 and the spread 12 of absorbent material.

In the embodiment shown in Figure 4, the first spread 6 of absorbent material comprises the first absorbent polymer material SAP1, whereas the second spread 12 of absorbent material comprises the second absorbent polymer material SAP2.

Preferably, each cell 10 contains the same quantity of absorbent material SAP1 and of absorbent material SAP2.

The permeable inner layer 2 is positioned on the layer NW2 to define the outer structure of the absorbent sanitary article 1.

With reference to Figure 3, the layer NW1 is located on the impermeable outer layer 3.

The layer 7 of high loft non-woven fabric is located on the layer NW1.

In one embodiment, the pad 4 comprises an adhesive layer 11 between the layer NW1 and the layer 7.

The spread 6 of absorbent polymer material is located on the layer 7 of non-woven fabric.

In one embodiment, the pad 4 comprises an adhesive layer 14 between the layer 7 of non-woven fabric and the spread 6 of absorbent polymer material.

The layer NW2 is located on the spread 6 of absorbent polymer material and the permeable inner layer 2 is positioned on the layer NW2.

In one embodiment, the pad 4 comprises an adhesive layer 11 b between the layer NW2 of non-woven fabric and the spread 6 of absorbent polymer material.

With reference in particular to Figures 1, 3, 4, it may be observed that, especially when the layer NW2 is located on the spread 6, 13 of absorbent material SAP1 and/or SAP2, the absorbent sanitary article 1 comprises a further layer 15 of non-woven fabric illustrated, for simplicity, only in Figure 1.

The layer 15 is thus interposed between the pad 4 and the permeable inner layer 2.

In one embodiment, the layer 15 is glued to the layer NW2 and the pad 4 comprises an adhesive layer 16 between the layer NW2 and the layer 15 which is glued to the non-woven fabric NW2 on the side opposite the core 5.

In the preferred embodiment, the layer 15 is in the form of pre-cut pieces which are glued to the non-woven fabric NW2 on the side opposite the core 5.

Advantageously, the layer 15 makes the article 1 more comfortable because it defines a relatively softer layer between the absorbent material and the body of a wearer not illustrated.

The layer 15 of non-woven fabric is preferably similar to the layer 7 whose properties apply also to the layer 15.

Thus, the layer 15, too, is preferably made of high loft non-woven fabric.

It should be noted that the adhesive layers 11, 11 a, 11 b, 14, 16 meet process requirements in that they keep the different layers of the pad 4 in position during the assembly thereof.

In addition to the above mentioned basic components, absorbent articles comprise further accessory components, such as, for example, elastic bands, faecal barriers and side tabs, which vary the complexity of the structure and production process of the absorbent articles to different degrees and which are not described here because they do not form part of this invention.

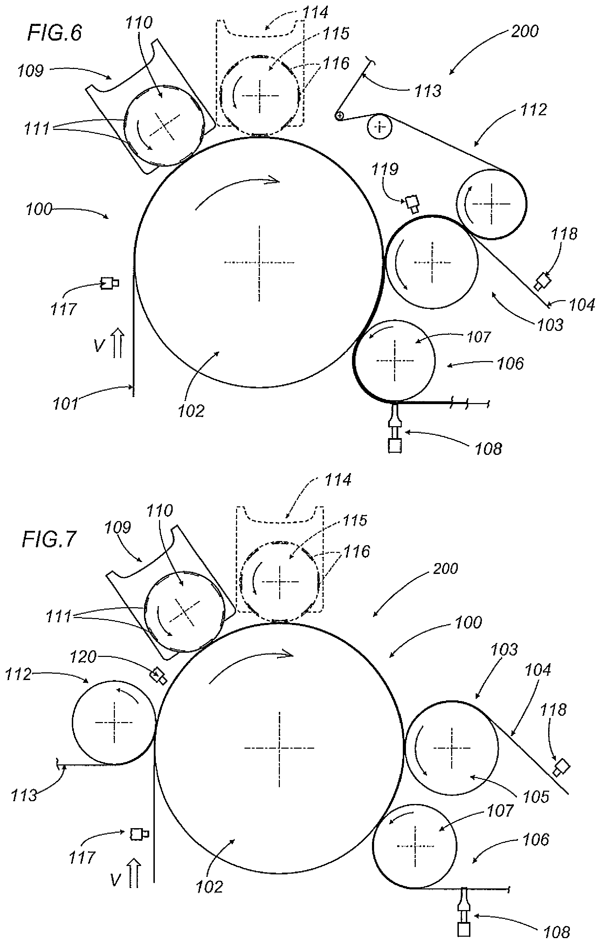

With reference to Figures 6 to 9, the numeral 200 denotes in its entirety a machine for making absorbent sanitary articles 1.

The description of the machine 200 is limited to the parts necessary for understanding this invention and, in particular, to the technical features which distinguish the different embodiments of it from each other, it being understood that the other features are the same for all the embodiments and denoted by the same reference numerals.

The machine 200 comprises a line, of substantially known type and not illustrated, for feeding at least one continuous web of impermeable material used to make the impermeable outer layer 3.

The machine 200 comprises a forming unit 100 for making the absorbent pads 4 to form a succession of the pads 4 and to associate them with the continuous web of impermeable material.

The unit 100 comprises a system for feeding a first web 101 of non-woven fabric in a feed direction V along a path P for defining the first layer NW1 of non-woven fabric.

The feed system comprises a suction roller 102 for holding down and feeding at least the first web 101 of non-woven fabric and rotatable in a clockwise direction.

The roller or drum 102 uses suction to hold down the web 101 and, preferably, the further layers of the article 1 subsequently placed on the web 101, as will become clearer as this description continues.

The unit 100 comprises a station 103 for placing a second web 104 of non-woven fabric on the first web 101 of non-woven fabric to define the second layer NW2 of non-woven fabric of the pad 4.

The station 103 comprises a roller 105 for transferring the second web 104 and tangent to the suction roller 102.

The roller 105 is preferably rotatable in anticlockwise direction.

The unit 100 comprises a station 106 for joining, in particular sealing, the second web 104 to the first web 101 so as to close the layers NW1 and NW2 on the cores 5 of the pads 4.

The joining station 106 comprises a contact roller 107, for example rotatable in anticlockwise direction, tangent to the suction roller 102 and located downstream of the transfer roller 105 relative to the feed direction V of the absorbent pads 4.

The roller 107 has on its outside surface a pattern of the seals 9, 9a.

The joining station 106 comprises a sonotrode 108, of substantially known type, for making the seals 9, 9a and facing the outside surface of the roller 107.

The sonotrode 108 acts on the contact roller 107 and, more specifically, makes the seals 9, 9a based on the predetermined pattern.

In an alternative embodiment not illustrated, the roller 105 itself acts as a contact roller, that is to say, it substitutes the roller 107, and the sonotrode 108 acts directly on the roller 105.

In a further embodiment not illustrated, the roller 102 is the contact roller, that is to say, it has the pattern of seals on its outside surface and the sonotrode 108 is a rotary sonotrode tangent to the roller 102. Advantageously, this embodiment comprises two sequentially rotating sonotrodes.

The unit 100 comprises a first station 109 for laying out the first 6 of absorbent polymer material on the web 101 of non-woven fabric.

The station 109 is operatively positioned upstream of the first placing station 103 relative to the first feed direction V.

The station 109 comprises a laying-out roller 110 which is rotatable, for example in anticlockwise direction, and which is substantially tangent to the suction roller 102.

The roller 110 is provided, on its outside cylindrical surface, with a plurality of pockets 111, each feeding and placing along the path P a portion of the absorbent material SAP1 and/or SAP2 of the first spread 6 of superabsorbent polymer material.

The forming unit 100 comprises a second station 112 for placing a third web 113 of non-woven fabric on the first web 101 of non-woven fabric to define the layer 7 of non-woven fabric.

As illustrated, the station 112 is located upstream of the station 103 relative to the feed direction V.

In the embodiment of Figure 6, the station 112 is located downstream of the laying-out station 109 relative to the feed direction V.

More specifically, the station 112 applies the web 113 to the web 104 in such a way that the web 113 will be on top of the web 101 in the pad 4.

In other words, the station 112 is located downstream of the laying-out station 109 in the sense that it acts on the web 104 to apply the web 113 thereto, and the web 104 which, as mentioned above will define the layer NW2 of the pad 4, is applied to the web 101 after the material has been laid out at the station 109.

This configuration makes it possible to obtain the article 1 of Figure 2. Preferably, the forming unit 100 comprises a second station 114, shown by the dashed lines, for laying out the second spread 12 of absorbent polymer material.

The second station 114 is located downstream of the first laying-out station 109 and upstream of the second placing station 112, relative to the feed direction V.

As illustrated, the second laying-out station 114 comprises a laying-out roller 115 substantially tangent to the suction roller 102 and rotatable, for example, in anticlockwise direction.

The roller 115 is provided, on its outside cylindrical surface, with a plurality of pockets 116, each feeding and placing along the path P a portion of the absorbent material SAP1 and/or SAP2 of the second spread 12 of superabsorbent polymer material.

The forming unit 100 comprises an adhesive dispenser 117 operating on the web 101 upstream of the laying-out station 109 to apply the layer 11 on the web 101 of non-woven fabric.

Preferably, the forming unit 100 comprises a second adhesive dispenser 118 operating at the first placing station 103 to spread the adhesive 11 b on the second web 102.

As already mentioned, the adhesive 11b keeps the web 113 in position relative to the web 104 and the unit 100 comprises an adhesive dispenser 119 for applying the adhesive 11 a to the layer 113 once the latter has been glued to the web 104.

In practice, the dispenser 119 restores the layer of adhesive which will go on top of the absorbent material.

With reference to Figure 7, in the embodiment illustrated, which forms the pad 4 shown in Figure 3, the station 112 for placing the web 113 is located upstream of the first laying-out station 109 and downstream of the adhesive dispenser 117, relative to the feed direction V.

Preferably, the unit 100 comprises an adhesive dispenser 120 for applying the adhesive 11 to the layer 113 once the latter has been glued to the web 101.

In practice, the dispenser 120 restores the layer of adhesive which will go under the absorbent material.

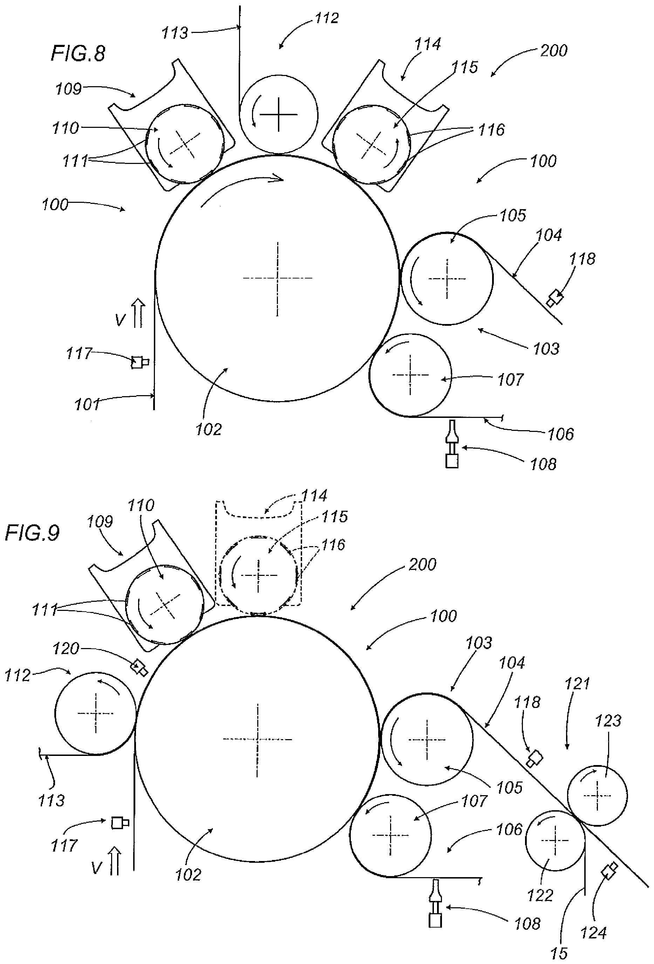

With reference to Figure 8, in the embodiment illustrated, which forms the pad 4 shown in Figure 4, the second station 114 for laying out the second spread 12 of absorbent material is located downstream of the station 112 for placing the web 113 and upstream of the station 103 for placing the web 104, relative to the feed direction V.

With reference to Figure 9, in the embodiment where the high loft layer 15 is to be provided on the outside of the pad 4, between the layer NW2 and the permeable layer 2, the unit 100 for forming the pad 4 comprises a station 121 for applying the layer 15 to the web 104.

The application station 121 comprises a roller 122 for feeding the layer 15 of high loft non-woven fabric and a contact roller 123 tangent to the roller 122 at the web 104.

The station 121 comprises a dispenser 124 for spreading the adhesive 16 on the web 104, upstream of the rollers 122 and 123 relative to the feed direction of the web 102 in such a way that the rollers 122 and 123 cause the web 104 to be joined to the layer 15.

The machine 200 comprises a line, of substantially known type and not illustrated, for feeding and applying at least one continuous web of permeable material used to make the permeable inner layer 2.

The pads 4 are interposed between the outer layer 3 and the inner layer 2. The invention described brings important advantages.

The layer 7 of loft or high loft non-woven fabric helps hold the core 5 together better, especially if the seals 9, 9a are torn and, in practice, makes up for the absence of cellulose fibre, or fluff, in the absorbent core 5.

Thanks to the production process, the absorbent material SAP1, SAP2 is embedded in the layer 7 and is thus made more stable.

The layer 15 on the outside of the core 5 makes the article 1 more comfortable, especially in the case where the absorbent material SAP1, SAP2 is directed towards the wearer's skin.

The adhesive layers limit the migration of the absorbent material both when the absorbent material is dry and during the formation of the pad 4.

## Claims

1. A machine for making absorbent sanitary articles (1) having a main longitudinal direction of extension (D) and comprising
- a permeable inner layer (2);
- an impermeable outer layer (3);
- an absorbent pad (4) interposed between the inner layer (2) and the outer layer (3) and comprising:
- a first layer (NW1) of non-woven fabric;
- a second layer (NW2) of non-woven fabric partly joined to the first layer (NW1) of non-woven fabric;
- an absorbent core (5) interposed between the first and second layers (NW1, NW2) of non-woven fabric, the absorbent core (5) comprising a spread (6) of absorbent polymer material (SAP1, SAP2);
- a plurality of seals (9, 9a) for connecting the first layer (NW1) of non-woven fabric to the second layer (NW2) of non-woven fabric;
- a third layer (7) of non-woven fabric interposed between the first and second layers (NW1, NW2) of non-woven fabric, the machine comprising a line for feeding at least one continuous web of impermeable material for defining the impermeable outer layer (3), a line for feeding at least one continuous web of permeable material for defining the permeable inner layer (2) and at least one unit (100) for forming the absorbent pads (4) to form a succession of the absorbent pads (4) and to associate the absorbent pads (4) with the continuous web of impermeable material, the forming unit (100) comprising a system for feeding a first web (101) of non-woven fabric in a feed direction (V) along a path (P) for defining the first layer (NW1) of non-woven fabric, a first station (103) for placing a second web (104) of non-woven fabric on the first web (101) of non-woven fabric for defining the second layer (NW2) of non-woven fabric, a station (106) for joining the second web (104) of non-woven fabric to the first web (101) of non-woven fabric, a first station (109) for laying out the first spread (6) of absorbent polymer material on the first web (101) of non-woven fabric and being operatively positioned upstream of the first placing station (103) relative to the first feed direction (V), the machine being **characterized in that** the forming unit (100) comprises a second station (112) for placing a third web (113) of non-woven fabric between the first web (101) of non-woven fabric and the second web (104) of non-woven fabric which defines the third layer (7) of non-woven fabric and which operates upstream of the first placing station (103) relative to the feed direction (V).

2. The machine according to claim 1, wherein the second placing station (112) is located downstream of the first laying-out station (109) relative to the feed direction.

3. The machine according to claim 2, wherein the forming unit (100) comprises a second station (114) for laying out a second spread (12) of absorbent polymer material and being located downstream of the second placing station (112) and downstream of the first placing station (103) relative to the feed direction (V).

4. The machine according to claim 2, wherein the forming unit (100) comprises a second station (112) for laying out a second spread (12) of absorbent polymer material and being located downstream of the first laying-out station (109) and upstream of the first placing station (103) relative to the feed direction (V).

5. The machine according to any one of claims 1 to 4, wherein the forming unit (100) comprises a first adhesive dispenser (117) operating at the feed system to spread an adhesive layer (11) between the first layer (NW1) of non-woven fabric and the third layer (7) of non-woven fabric or between the first layer (NW1) of non-woven fabric and the spread (6) of absorbent polymer material, the first adhesive dispenser (117) being located upstream of the first laying-out station (109) relative to the feed direction.

6. The machine according to any one of claims 1 to 5, wherein the forming unit (100) comprises a second adhesive dispenser (118) operating at the first placing station (103) to spread an adhesive layer (11b) between the first spread (6) of absorbent polymer material and the second layer (NW2) of non-woven fabric or between a second spread (12) of absorbent polymer material and the second layer (NW2) of non-woven fabric, or between the third layer (7) of non-woven fabric and the second layer (NW2) of non-woven fabric.

7. The machine according to claim 5, wherein the second placing station (112) is located upstream of the first laying-out station (109) and downstream of the first adhesive dispenser (117) relative to the feed direction.

8. The machine according to claim 7, wherein the forming unit comprises a third adhesive dispenser (120) which is located downstream of the second placing station (112) and upstream or downstream of the first laying-out station (109) and which spreads an adhesive layer (14) between the third layer (7) of non-woven fabric and the spread (6) of absorbent polymer material.

9. The machine according to any one of claims 1 to 8, wherein the feed system comprises a suction roller (102) for holding down and feeding at least the first web (101) of non-woven fabric.

10. The machine according to claim 9, wherein the first laying-out station (109) and/or the second laying-out station (114) comprise a laying-out roller (110, 115) which is substantially tangent to the suction roller (102) and which is provided on its outside cylindrical surface with a plurality of pockets (111, 116), each feeding and placing along the path (P) a portion of the first and/or second spread (6, 12) of absorbent polymer material.

11. The machine according to claim 9 or 10, wherein the first station (103) for placing the second web (104) of non-woven fabric comprises a roller (105) which transfers the second web (104) of non-woven fabric and which is tangent to the suction roller (102) and the joining station (106) comprises a contact roller (107) tangent to the suction roller (102) and located downstream of the transfer roller (105) relative to the feed direction (V) of the absorbent pads (4), the joining station (106) comprising at least one sonotrode (108) for sealing the absorbent pads (4) and acting on the contact roller (107), the contact roller (107) having on its outside surface a predetermined pattern of the seals (9, 9a).

12. The machine according to any one of claims 1 to 11, wherein the forming unit (100) comprises a station (121) for applying a fourth layer (15) of non-woven fabric to the second web (104) of non-woven fabric, the application station (121) comprising a roller (122) for feeding the fourth layer (15) of non-woven fabric and tangent to the second web (104) of non-woven fabric, the application station (121) comprising an adhesive dispenser (124) operating on the second web (104) of non-woven fabric and located upstream of the roller (122) for feeding the fourth layer (15) of non-woven relative to the feed direction of the second web (104) of non-woven fabric.

13. The machine according to claim 9 or 10, wherein the first station (103) for placing the second web (104) of non-woven fabric comprises a roller (105) which transfers the second web (104) of non-woven fabric and which is tangent to the suction roller (102), the joining station (106) comprising the transfer roller (105), the transfer roller comprising on its outside cylindrical surface a predetermined pattern of the seals and defining a contact roller, the joining station (106) comprising at least one sonotrode (108) for sealing the absorbent pads (4) and acting on the contact roller (107).

14. The machine according to claim 9 or 10, wherein the joining station (106) comprises the suction roller (102), the suction roller comprising on its outside cylindrical surface a predetermined pattern of the seals and defining a contact roller, the joining station (106) comprising at least one rotary sonotrode for sealing the absorbent pads (4) and acting on the suction roller (102).

## Patentansprüche

1. Maschine zur Herstellung absorbierender Hygieneartikel (1), aufweisend eine Hauptlängsausdehnungsrichtung (D) und umfassend
- eine durchlässige Innenschicht (2);
- eine undurchlässige Außenschicht (3);
- eine absorbierende Einlage (4), die zwischen der durchlässigen Innenschicht (2) und der undurchlässigen Außenschicht (3) eingesetzt ist, und umfassend:
- eine erste Schicht (NW1) aus Vliesstoff;
- eine zweite Schicht (NW2) aus Vliesstoff, die teilweise mit der ersten Schicht (NW1) aus Vliesstoff zusammengefügt ist;
- einen absorbierenden Kern (5), der zwischen der ersten und der zweiten Schicht (NW1, NW2) aus Vliesstoff eingefügt ist, wobei der absorbierende Kern (5) eine Spanne (6) aus absorbierendem Polymermaterial (SAP1, SAP2) umfasst;
- eine Vielzahl an Siegeln (9, 9a), um die erste Schicht (NW1) aus Vliesstoff mit der zweiten Schicht (NW2) aus Vliesstoff zu verbinden;
- eine dritte Schicht (7) aus Vliesstoff, die zwischen der ersten und der zweiten Schicht (NW1, NW2) aus Vliesstoff eingesetzt ist, wobei die Maschine eine Anlage zum Zuführen von mindestens einer durchgehenden Bahn aus undurchlässigem Material zum Definieren der undurchlässigen Außenschicht (3), eine Anlage zum Zuführen von mindestens einer durchgehenden Bahn aus durchlässigem Material zum Definieren der durchlässigen Innenschicht (2) und mindestens eine Einheit (100) zum Formen der absorbierenden Einlagen (4) zum Formen einer Abfolge an absorbierenden Einlagen (4) und zum Assoziieren der absorbierenden Einlagen (4) mit der durchgehenden Bahn aus undurchlässigem Material umfasst, wobei die Formungseinheit (100) ein System zum Zuführen einer ersten Bahn (101) aus Vliesstoff in einer Zuführungsrichtung (V) entlang eines Wegs (P) zum Definieren der ersten Schicht (NW1) aus Vliesstoff, eine erste Station (103) zum Platzieren einer zweiten Bahn (104) aus Vliesstoff auf der ersten Bahn (101) aus Vliesstoff zum Definieren der zweiten Schicht (NW2) aus Vliesstoff, eine Station (106) zum Zusammenfügen der zweiten Bahn (104) aus Vliesstoff mit der ersten Bahn (101) aus Vliesstoff, eine erste Station (109) zum Ausbringen der ersten Spanne (6) aus absorbierendem Polymermaterial auf der ersten Bahn (101) aus Vliesstoff umfasst, betriebswirksam positioniert vor der ersten Platzierungsstation (103) relativ zur ersten Zuführungsrichtung (V), wobei die Maschine **dadurch gekennzeichnet ist, dass** die Formungseinheit (100) eine zweite Station (112) umfasst, um eine dritte Bahn (113) aus Vliesstoff zwischen der ersten Bahn (101) aus Vliesstoff und der zweiten Bahn (104) aus Vliesstoff zu platzieren, die die dritte Schicht (7) aus Vliesstoff definiert und die vor der ersten Platzierungsstation (103) relativ zur Zuführungsrichtung (V) arbeitet.

2. Maschine nach Anspruch 1, wobei die zweite Platzierungsstation (112) nach der ersten Ausbringungsstation (109) relativ zur Zuführungsrichtung angeordnet ist.

3. Maschine nach Anspruch 2, wobei die Formungseinheit (100) eine zweite Station (114) zum Ausbringen einer zweiten Spanne (12) aus absorbierendem Polymermaterial umfasst und nach der zweiten Platzierungsstation (112) und nach der ersten Platzierungsstation (103) relativ zur Zuführungsrichtung (V) angeordnet ist.

4. Maschine nach Anspruch 2, wobei die Formungseinheit (100) eine zweite Station (112) zum Ausbringen einer zweiten Spanne (12) aus absorbierendem Polymermaterial umfasst und nach der ersten Platzierungsstation (109) und vor der ersten Platzierungsstation (103) relativ zur Zuführungsrichtung (V) angeordnet ist.

5. Maschine nach einem der Ansprüche 1 bis 4, wobei die Formungseinheit (100) eine erste Haftmittelausgabevorrichtung (117) umfasst, die am Zuführungssystem arbeitet, um eine Haftmittelschicht (11) zwischen der ersten Schicht (NW1) aus Vliesstoff und der dritten Schicht (7) aus Vliesstoff oder zwischen der ersten Schicht (NW1) aus Vliesstoff und der Spanne (6) aus absorbierendem Polymermaterial zu verteilen, wobei die erste Haftmittelausgabevorrichtung (117) vor der ersten Ausbringungsstation (109) relativ zur Zuführungsrichtung angeordnet ist.

6. Maschine nach einem der Ansprüche 1 bis 5, wobei die Formungseinheit (100) eine zweite Haftmittelausgabevorrichtung (118) umfasst, die an der ersten Platzierungsstation (103) arbeitet, um eine Haftmittelschicht (11b) zwischen der ersten Spanne (6) aus absorbierendem Polymermaterial und der zweiten Schicht (NW2) aus Vliesstoff oder zwischen einer zweiten Spanne (12) aus absorbierendem Polymermaterial und der zweiten Schicht (NW2) aus Vliesstoff oder zwischen der dritten Schicht (7) aus Vliesstoff und der zweiten Schicht (NW2) aus Vliesstoff zu verteilen.

7. Maschine nach Anspruch 5, wobei die zweite Platzierungsstation (112) vor der ersten Ausbringungsstation (109) und nach der ersten Haftmittelausgabevorrichtung (117) relativ zur Zuführungsrichtung angeordnet ist.

8. Maschine nach Anspruch 7, wobei die Formungseinheit eine dritte Haftmittelausgabevorrichtung (120) umfasst, die nach der zweiten Platzierungsstation (112) und vor oder nach der ersten Ausbringungsstation (109 angeordnet ist und die eine Haftmittelschicht (14) zwischen der dritten Schicht (7) aus Vliesstoff und der Spanne (6) aus absorbierendem Polymermaterial verteilt.

9. Maschine nach einem der Ansprüche 1 bis 8, wobei das Zuführungssystem eine Saugwalze (102) umfasst, um zumindest die erste Bahn (101) aus Vliesstoff nach unten zu halten und zuzuführen.

10. Maschine nach Anspruch 9, wobei die erste Ausbringungsstation (109) und/oder die zweite Ausbringungsstation (114) eine Ausbringungswalze (110, 115) umfassen, die die Saugwalze (102) im Wesentlichen berührt und die an ihrer zylindrischen außenseitigen Oberfläche mit einer Vielzahl an Taschen (111, 116) versehen ist, von denen eine jede einen Abschnitt der ersten und/oder zweiten Spanne (6, 12) aus absorbierendem Polymermaterial zuführt und entlang des Wegs (P) platziert.

11. Maschine nach Anspruch 9 oder 10, wobei die erste Station (103) zum Platzieren der zweiten Bahn (104) aus Vliesstoff eine Walze (105) umfasst, die die zweite Bahn (104) aus Vliesstoff transferiert und die die zweite Saugwalze (102) berührt, und die Zusammenfügungsstation (106) eine Kontaktwalze (107) umfasst, die die Saugwalze (102) berührt und nach der Transferwalze (105) relativ zur Zuführungsrichtung (V) der absorbierenden Einlagen (4) angeordnet ist, wobei die Zusammenfügungsstation (106) mindestens eine Sonotrode (108) umfasst, um die absorbierenden Einlagen (4) zu siegeln und auf die Kontaktwalze (107) zu wirken, wobei die Kontaktwalze (107) an ihrer außenseitigen Oberfläche ein vorgegebenes Muster an Siegeln (9, 9a) aufweist.

12. Maschine nach einem der Ansprüche 1 bis 11, wobei die Formungseinheit (100) eine Station (121) zum Anbringen einer vierten Schicht (15) aus Vliesstoff an der zweiten Bahn (104) aus Vliesstoff umfasst, wobei die Anbringungsstation (121) eine Walze (122) zum Zuführen der vierten Schicht (15) aus Vliesstoff umfasst, die die zweite Bahn (104) aus Vliesstoff berührt, wobei die Anbringungsstation (121) eine Haftmittelausgabevorrichtung (124) umfasst, die auf der zweiten Bahn (104) aus Vliesstoff arbeitet und vor der Walze (121) zum Zuführen der vierten Schicht (15) aus Vliesstoff relativ zur Zuführungsrichtung der zweiten Bahn (104) aus Vliesstoff angeordnet ist.

13. Maschine nach Anspruch 9 oder 10, wobei die erste Station (103) zum Platzieren der zweiten Bahn (104) aus Vliesstoff eine Walze (105) umfasst, die die zweite Bahn (104) aus Vliesstoff transferiert und die Saugwalze (102) berührt, wobei die Zusammenfügungsstation (106) die Transferwalze (105) umfasst, wobei die Transferwalze an ihrer außenseitigen zylindrischen Oberfläche ein vorgegebenes Muster an Siegeln umfasst und eine Kontaktwalze definiert, wobei die Zusammenfügungsstation (106) mindestens eine Sonotrode (108) umfasst, um die absorbierenden Einlagen (4) zu siegeln und auf die Kontaktwalze (107) zu wirken.

14. Maschine nach Anspruch 9 oder 10, wobei die Zusammenfügungsstation (106) die Saugwalze (102) umfasst, wobei die Saugwalze an ihrer außenseitigen zylindrischen Oberfläche ein vorgegebenes Muster an Siegeln umfasst und eine Kontaktwalze definiert, wobei die Zusammenfügungsstation (106) mindestens eine sich drehende Sonotrode umfasst, um die absorbierenden Einlagen (4) zu siegeln und auf die Saugwalze (102) zu wirken.

## Revendications

1. Machine pour fabriquer des articles hygiéniques absorbants (1) disposant d'une direction d'extension (D) longitudinale principale et comprenant
- une couche interne perméable (2) ;
- une couche externe imperméable (3) ;
- un tampon absorbant (4) interposé entre la couche interne (2) et la couche externe (3) et comprenant :
- une première couche (NW1) de tissu non tissé ;
- une seconde couche (NW2) de tissu non tissé partiellement reliée à la première couche (NW1) de tissu non tissé ;
- un coeur absorbant (5) interposé entre les première et seconde couches (NW1, NW2) de tissu non tissé, le coeur absorbant (5) comprenant un étalement (6) d'un polymère absorbant (SAP1, SAP2) ;
- une pluralité de jointures (9, 9a) servant à relier la première couche (NW1) de tissu non tissé à la seconde couche (NW2) de tissu non tissé ;
- une troisième couche (7) de tissu non tissé interposée entre les première et seconde couches (NW1, NW2) de tissu non tissé, la machine comprenant une ligne servant à alimenter au moins une bande continue de matériau imperméable pour définir la couche externe imperméable (3), une ligne pour alimenter au moins une bande continue de matériau perméable pour définir la couche interne perméable (2) et au moins une unité (100) pour former les tampons absorbants (4) pour former une succession de tampons absorbants (4) et pour associer les tampons absorbants (4) à la bande continue de matériau imperméable, l'unité de formation (100) comprenant un système servant à alimenter une première bande (101) de tissu non tissé dans une direction d'alimentation (V) le long d'un parcours (P) pour définir la première couche (NW1) de tissu non tissé, un premier poste (103) servant à positionner une seconde bande (104) de tissu non tissé sur la première bande (101) de tissu non tissé pour définir la seconde couche (NW2) de tissu non tissé, un poste (106) servant à assembler la seconde bande (104) de tissu non tissé à la première bande (101) de tissu non tissé, un premier poste (109) servant à déposer le premier étalement (6) de polymère absorbant sur la première bande (101) de tissu non tissé et étant fonctionnellement positionné en amont du premier poste de positionnement (103) par rapport à la première direction d'alimentation (V), la machine étant **caractérisée en ce que** l'unité de formation (100) comprend un second poste (112) servant à positionner une troisième bande (113) de tissu non tissé entre la première bande (101) de tissu non tissé et la seconde bande (104) de tissu non tissé qui définit la troisième couche (7) de tissu non tissé et qui fonctionne en amont du premier poste de positionnement (103) par rapport à la direction d'alimentation (V).

2. Machine selon la revendication 1, dans laquelle le second poste de positionnement (112) est situé en aval du premier poste de déposition (109) par rapport à la direction d'alimentation.

3. Machine selon la revendication 2, dans laquelle l'unité de formation (100) comprend un second poste (114) servant à déposer un second étalement (12) de polymère absorbant et étant situé en aval du second poste de positionnement (112) et en aval du premier poste de positionnement (103) par rapport à la direction d'alimentation (V).

4. Machine selon la revendication 2, dans laquelle l'unité de formation (100) comprend un second poste (112) servant à déposer un second étalement (12) de polymère absorbant et étant situé en aval du premier poste de déposition (109) et en amont du premier poste de positionnement (103) par rapport à la direction d'alimentation (V).

5. Machine selon l'une quelconque des revendications de 1 à 4, dans laquelle l'unité de formation (100) comprend un premier distributeur d'adhésif (117) fonctionnant au niveau du système d'alimentation pour étaler une couche adhésive (11) entre la première couche (NW1) de tissu non tissé et la troisième couche (7) de tissu non tissé ou entre la première couche (NW1) de tissu non tissé et l'étalement (6) de polymère absorbant, le premier distributeur d'adhésif (117) étant situé en amont du premier poste de déposition (109) par rapport à la direction d'alimentation.

6. Machine selon l'une quelconque des revendications de 1 à 5, dans laquelle l'unité de formation (100) comprend un second distributeur d'adhésif (118) fonctionnant au niveau du premier poste de positionnement (103) pour étaler une couche adhésive (11b) entre le premier étalement (6) de polymère absorbant et la seconde couche (NW2) de tissu non tissé ou entre un second étalement (12) de polymère absorbant et la seconde couche (NW2) de tissu non tissé ou entre la troisième couche (7) de tissu non tissé et la seconde couche (NW2) de tissu non tissé.

7. Machine selon la revendication 5, dans laquelle le second poste de positionnement (112) est situé en amont du premier poste de déposition (109) et en aval du premier distributeur d'adhésif (117) par rapport à la direction d'alimentation.

8. Machine selon la revendication 7, dans laquelle l'unité de formation comprend un troisième distributeur d'adhésif (120), étant situé en aval du second poste de positionnement (112) et en amont ou en aval du premier poste de déposition (109), et qui étale une couche adhésive (14) entre la troisième couche (7) de tissu non tissé et l'étalement (6) de polymère absorbant.

9. Machine selon l'une quelconque des revendications de 1 à 8, dans laquelle le système d'alimentation comprend un rouleau d'aspiration (102) servant à maintenir et à alimenter au moins la première bande (101) de tissu non tissé.

10. Machine selon la revendication 9, dans laquelle le premier poste de déposition (109) et/ou le second poste de déposition (114) comprend/comprennent un rouleau de déposition (110, 115) étant substantiellement tangent au rouleau d'aspiration (102) et étant pourvu sur sa surface cylindrique extérieure d'une pluralité de poches (111, 116), chacune alimentant et plaçant le long du parcours (P) une partie du premier et/ou du second étalement (6, 12) de polymère absorbant.

11. Machine selon les revendications 9 ou 10, dans laquelle le premier poste (103) servant à positionner la seconde bande (104) de tissu non tissé comprend un rouleau (105) qui transfère la seconde bande (104) de tissu non tissé et étant tangent au rouleau d'aspiration (102) et le poste d'assemblage (106) comprend un rouleau de contact (107) tangent au rouleau d'aspiration (102) et situé en aval du rouleau de transfert (105) par rapport à la direction d'alimentation (V) des tampons absorbants (4), le poste d'assemblage (106) comprenant au moins une sonotrode (108) pour sceller les tampons absorbants (4) et agir sur le rouleau de contact (107), le rouleau de contact (107) comportant sur sa surface extérieure un motif prédéfini de jointures (9, 9a).

12. Machine selon l'une quelconque des revendications de 1 à 11, dans laquelle l'unité de formation (100) comprend un poste (121) servant à appliquer une quatrième couche (15) de tissu non tissé à la seconde bande (104) de tissu non tissé, le poste d'application (121) comprenant un rouleau (122) servant à alimenter la quatrième couche (15) de tissu non tissé et tangent à la seconde bande (104) de tissu non tissé, le poste d'application (121) comprenant un distributeur d'adhésif (124) fonctionnant sur la seconde bande (104) de tissu non tissé et situé en amont du rouleau (122) pour alimenter la quatrième couche (15) de tissu non tissé par rapport à la direction d'alimentation de la seconde bande (104) de tissu non tissé.

13. Machine selon les revendications 9 ou 10, dans laquelle le premier poste (103) servant à positionner la seconde bande (104) de tissu non tissé comprend un rouleau (105) qui transfère la seconde bande (104) de tissu non tissé et étant tangent au rouleau d'aspiration (102), le poste d'assemblage (106) comprenant le rouleau de transfert (105), le rouleau de transfert comprenant au niveau de sa surface cylindrique extérieure un motif prédéfini de jointures et définissant un rouleau de contact, le poste d'assemblage (106) comprenant au moins une sonotrode (108) pour sceller les tampons absorbants (4) et agir sur le rouleau de contact (107).

14. Machine selon les revendications 9 ou 10, dans laquelle le poste d'assemblage (106) comprend le rouleau d'aspiration (102), le rouleau d'aspiration comprenant sur sa surface cylindrique extérieure un motif prédéfini de jointures et définissant un rouleau de contact, le poste d'assemblage (106) comprenant au moins une sonotrode rotative pour sceller les tampons absorbants (4) et agir sur le rouleau d'aspiration (102).
